# EUROPEAN PATENT APPLICATION

(11) **EP 3 081 245 A1**
(43) Date of publication of application: **19.10.2016**
(21) Application number: 14869450.8
(22) Date of filing: 04.12.2014
(51) Int. Cl.: A61M 1/08

(54) **CONSTANT PRESSURE CUPPING DEVICE**

(30) Priority: 13.12.2013 KR 20130155669
(71) Applicant: Korea Institute of Oriental Medicine, Daejeon 305-811 (KR)
(72) Inventor: KWON, O Sang, Iksan-si Jeollabuk-do 570-758 (KR); PARK, Ji Eun, Daejeon 305-761 (KR); RYU, Yeon Hee, Daejeon 305-761 (KR)
(74) Representative: Merryweather, Colin Henry
(86) International application number: PCT/KR2014/011805
(87) International publication number: WO 2015/088181

(57) **Abstract**

A cupping device according to one embodiment comprises: a cupping cup which is adhered to a region requiring cupping therapy; an inlet port which communicates the inside and outside of the cupping cup; and a pressure controller which is capable of selectively opening and closing the inlet port, wherein the movement of the pressure controller allows or blocks air inflow into the cupping cup.

## Description

### Technical Field

The present invention relates to a cupping device and, more particularly, to a cupping device to maintain a pressure to be constant in a cupping cup.

### Background Art

In general, a cupping therapy is known as a therapeutic method of oriental medicine applied by adhering a cupping cup (also referred to as "cupping pot") of a cupping device to an affected area of a patient, creating a vacuum in the cupping cup, and forcibly filtering and resolving an extravasated blood including, for example, wastes and toxins in a capillary vessel to acquire an autologous serum proteome. Thus, the cupping therapy may be used to remove the extravasated blood.

The cupping therapy may be applied based on various methods of creating a vacuum in the cupping cup such as a fire cupping method, a water suction method, a suction cup method, and the like, for example.

The fire cupping method may involve firing with alcohol inside the cupping cup to create a negative pressure in the cupping cup in a vacuum state. The water suction method may involve heating the cupping cup with boiling water to create a negative pressure in the cupping cup in a vacuum state. The suction cup method may involve withdrawing an air from the cupping cup using a device such as a vacuum pump to create a negative pressure in the cupping cup in a vacuum state.

With popularization of the cupping therapy, research on the cupping device has been conducted in various ways. For example, Korean Patent Application No. 10-2010-0119343 filed on November 29, 2011 discloses a high frequency-based cupping device.

### Disclosure of Invention

### Technical Goals

An aspect of the present embodiment provides a cupping device to efficiently maintain a pressure to be constant in a cupping cup.

Another aspect of the present embodiment provides a cupping device to prevent an adverse effect such as blistering, bleeding, and the like that may occur due to an over-pressure in a cupping cup.

Another aspect of the present embodiment provides a cupping device including a pressure controller provided in a relatively simple structure to be easily detached or replaced from the cupping device.

### Technical solutions

According to an aspect of the present embodiment, there is provided a cupping device including a cupping cup attached to an area requiring a cupping therapy, an inlet configured to allow communication between an inside and an outside of the cupping cup, and a pressure controller configured to selectively open and close the inlet, wherein an air inflow into the cupping cup is allowed or blocked based on a movement of the pressure controller.

The cupping cup may include an inlet member and the pressure controller may be disposed in the inlet member.

The pressure controller may include a rod provided in a form of bar, a spring elastically deformed and configured to cover the rod, and a stopper attached to one end of the rod to open and close the inlet, wherein the rod and the stopper are configured to move in the inlet member based on a difference between an internal pressure and an external pressure of the cupping cup.

The inlet member may include a through-hole of a size that does not allow the spring and the stopper to move out of the inlet member and allows the rod to move out of the inlet member.

The inlet may be formed on a path along which the stopper moves in the inlet member so as to be open when the stopper passes by the inlet.

An elasticity of the spring may correspond to the difference between the internal pressure and the external pressure of the cupping cup, and a degree of deformation of the spring and a moving distance of the rod and the stopper change based on the difference.

The inlet member may open outward of the cupping cup to allow the air inflow.

According to another aspect of the present embodiment, there is also provided a pressure control device for controlling an internal pressure of a case, the device including an inlet member attached on an inner side of the case and formed to have one open side to communicate with an outside of the case, an inlet formed on one side of the inlet member, a stopper configured to move in the inlet member to open and close the inlet, and a spring disposed in the inlet member to be elastically deformed in response to a movement of the stopper.

The pressure control device may further include a rod formed to have one side attached to the stopper and externally enclosed by the spring, wherein a through-hole may be formed in the inlet member through which the rod moves out of the inlet member.

### Effects

According to an aspect of the present embodiment, it is possible to provide a cupping device to efficiently maintain a constant pressure in a cupping cup.

According to another aspect of the present embodiment, it is possible to prevent an adverse effect such as blistering, bleeding, and the like that may occur due to an over-pressure in a cupping cup.

According to still another aspect of the present embodiment, it is possible to provide a pressure controller in a relatively simple structure so as to be easily detached or replaced from a cupping device.

### Brief Description of Drawings

FIG. 1 illustrates a cupping device according to an example embodiment.
FIG. 2 illustrates a cupping device including a cupping cup having a relatively small difference between an internal pressure and an external pressure according to an example embodiment.
FIG. 3 illustrates a cupping device including a cupping cup having a relatively large difference between an internal pressure and an external pressure according to an example embodiment.

### Best Mode for Carrying Out the Invention

Reference will now be made in detail to embodiments of the present invention, examples of which are illustrated in the accompanying drawings, wherein like reference numerals refer to the like elements throughout. The embodiments are described below in order to explain the present invention by referring to the figures.

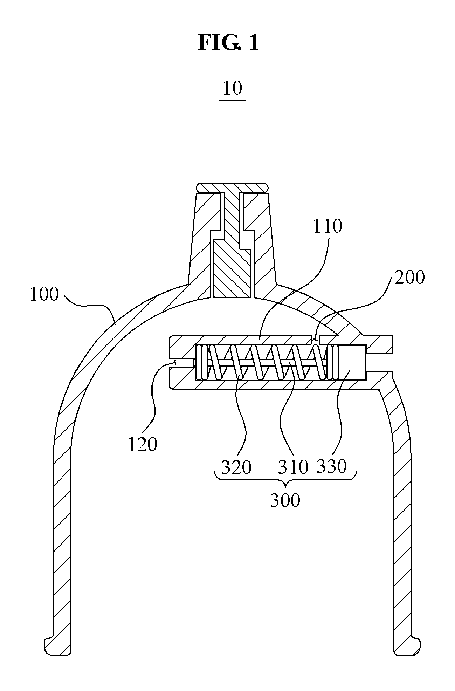

FIG. 1 illustrates a cupping device according to an example embodiment.

Referring to FIG. 1, a cupping device 10 may include a cupping cup 100, an inlet 200, and a pressure controller 300.

The cupping cup 100 may be provided in a shape of a hemisphere having an open lower portion. Also, the cupping cup 100 may be formed of various materials, for example, a silicone, a glass, and a plastic.

Although FIG. 1 illustrates that the cupping cup 100 is provided in the shape of the hemisphere as an example, the example is not to be taken as being limited thereto. Thus, any shape for applying a cupping therapy to a wide area is applicable to the cupping cup 100.

The cupping cup 100 may be disposed on an area of a body surface to perform the cupping therapy.

Specifically, the open lower portion of the cupping cup 100 may be attached to an affected area of a patient and a negative pressure may be applied to an inside of the cupping cup 10 such that the cupping therapy is performed.

The cupping cup 100 may have an inlet member 110.

The inlet member 110 may be formed to inwardly extend from a side of the cupping cup 100.

The inlet member 110 may have one side configured to be open and closed.

An external air may be flow into the inlet member 110 through the one side while the one side is open. For example, when the one side of is closed, the inlet member 110 may block the air from flowing into the cupping cup 100.

The inlet member 110 may be provided to arrange the pressure controller 300 as further discussed below and may also not be included in the cupping device 10. Thus, an inlet member may not be formed depending on an example.

Hereinafter, for example, the cupping cup 100 having the inlet member 110 including the inlet 200 and the pressure controller 300 will be described as an example with reference to FIG. 1.

The inlet 200 may be configured to communicate the inside and an outside of the cupping cup 100.

Specifically, the inlet 200 may be formed on one side of the inlet member 110. The inlet 200 may allow the external air to flow into the cupping cup 100 or block the external air from flowing into the cupping cup 100.

For example, when the inlet 200 is open, the inside of the cupping cup 100 may communicate with the outside through the inlet 200 such that the external air flows into the cupping cup 100.

Conversely, when the inlet 200 is closed, the inlet 200 may block the inside of the cupping cup 100 from the outside such that the external air is blocked from flowing into the cupping cup 100.

The pressure controller 300 may be disposed in the inlet member 110 to selectively open and close the inlet 200.

The pressure controller 300 may include a rod 310, a spring 320, and a stopper 330.

The rod 310 may be provided in a form of a bar.

Although FIG. 1 illustrates, but not limited to, the pressure controller 300 including the rod 310, the rod 310 may also not included in the pressure controller 300 depending on an example. In such example, the pressure controller 300 may include the spring 320 and the stopper 330.

The rod 310 may move out of the inlet member 110 via a through-hole 120 relative to the inlet member 110.

Specifically, when the rod 310 moves due to a difference between an internal pressure and an external pressure of the cupping cup 100, the rod 310 may move out of the inlet member 110.

In this example, the through-hole 120 may be formed in a contacting area between the inlet member 110 and an end portion of the rod 310. Through the through-hole 120, the rod 310 maybe exposed outward of the inlet member 110.

The spring 320 may be elastically deformed and configured to enclose the rod 310.

For example, an outer diameter of the spring 320 may correspond to a diameter of the inlet member 110, and a height of the spring 320 may correspond to a width of the inlet member 110.

Also, a modulus of elasticity of the spring 320 may be set based on a desired pressure of a user.

For example, when the same pressure is applied to the spring 320, a degree of deformation of the spring 320 may increase according to an increase in the modulus of elasticity of the spring 320. Likewise, in this example, the degree of deformation of the spring 320 may decrease according to a decrease in the modulus of elasticity of the spring 320.

As such, the modulus of elasticity of the spring 320 may be a significant factor of designing the pressure controller 300.

Also, based on the modulus of elasticity of the spring 320, a position of the inlet 200 may be changed relative to the inlet member 110.

The stopper 330 may be attached to one side of the rod 310.

The stopper 330 may include, but not limited to, a rubber material. The stopper 330 may include any material that allows the stopper 330 to move based on the difference between the internal pressure and the external pressure of the cupping cup 100 and the inlet 200 to be sealed.

As illustrated in FIG. 1, the stopper 330 may be attached to one end of the rod 310 so as to be located in a open side of the inlet member 110.

The stopper 330 may open and close the inlet 200.

For example, the stopper 330 may be disposed outward relative to the inlet 220 to inwardly move in the inlet member 110 of the cupping cup 100.

In this example, power for moving the stopper 330 may be based on the difference between the internal pressure and the external pressure of the cupping cup 100. A moving distance of the stopper 330 may increase according to an increase in the difference.

Likewise, the moving distance of the stopper 330 may decrease according to a decrease in the difference.

The rod 310 connected with the stopper 330 may move in response to a movement of the stopper 330.

For example, the rod 310 may be moved by a distance to which the stopper 330 moves based on the difference between the internal pressure and the external pressure of the cupping cup 100.

The spring 320 may be elastically deformed in response to the movement of the stopper 330 and the rod 310.

Specifically, the spring 320 may not move in the inlet member 110 and thus, a pressurized degree of the spring 320 may increase according to an increase in a distance to which the stopper 330 and the rod 310 inwardly move in the cupping cup 100.

Thus, power for elastically deforming the spring 320 may also be based on the difference between the internal pressure and the external pressure in the cupping cup 100.

Concisely, a degree of deformation of the spring 320 and a moving distance of the rod 310 and the stopper 330 may vary based on the difference between the internal pressure and the external pressure of the cupping cup 100.

For example, the degree of deformation of the spring 320 and the moving distance of the rod 310 and the stopper 330 may increase according to an increase in the difference between the internal pressure and the external pressure of the cupping cup 100.

Likewise, in such example, the degree of deformation of the spring 320 and the moving distance of the rod 310 and the stopper 330 may decrease according to a decrease in the difference between the internal pressure and the external pressure of the cupping cup 100.

As described in the foregoing explanation, the pressure controller 300 may be configured in a simple structure and thus, easily detached or replaced from the inlet member 110 of the cupping cup 100.

For example, in a process of replacing the spring 320 or the stopper 330 of the pressure controller 300, the pressure controller 300 may be easily detached from the inlet member 110.

The aforementioned pressure controller and inlet member may be included in a separate pressure control device to control an internal pressure of a case.

In this example, the pressure control device may be attached on an inner side of the case to control the internal pressure. Also, the pressure control device may include an inlet member having one open side to communicate with an outside of the case, an inlet formed on one side of the inlet member, a stopper configured to move in the inlet member to open and close the inlet, and a spring disposed in the inlet member and elastically deformed in response to a movement of the stopper.

Specifically, the inlet may be selectively open and closed based on a difference between the internal pressure and an external pressure of the case. For example, the inlet may be open when the difference is relatively large, and the inlet may be closed when the difference is relatively small.

The inlet member may further include a rod. The stopper may be attached to one end of the rod. The spring may enclose around the rod.

On one side of the inlet member, a through-hole may be formed such that the rod moves out of the inlet member.

A configuration of the cupping device 10 are described with reference to the foregoing explanation. An operation of the cupping device 10 will be described with reference to the following explanation.

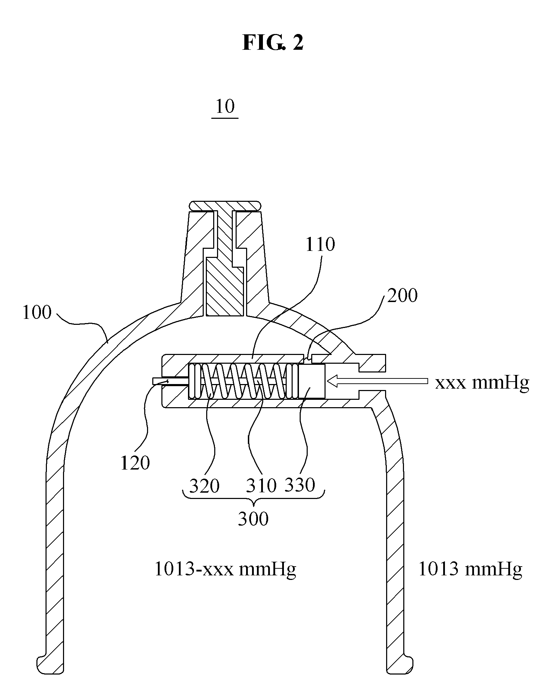

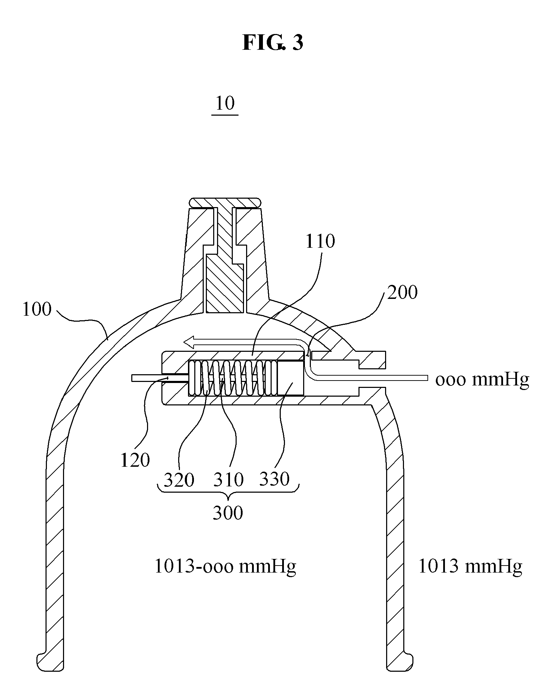

FIG. 2 illustrates a cupping device including a cupping cup having a relatively small difference between an internal pressure and an external pressure according to an example embodiment. FIG. 3 illustrates a cupping device including a cupping cup having a relatively large difference between an internal pressure and an external pressure according to an example embodiment.

In an example of FIG. 2, when a difference between an internal pressure and an external pressure of a cupping cup is relatively small, the cupping device 10 may perform an operation as described below.

For example, the external pressure of the cupping cup 100 may be 1013 millimeters of mercury (mmHg) and the internal pressure of the cupping cup 100 may be 1013 - xxx mmHg. In this example, the difference between the internal pressure and the external pressure may be xxx mmHg. Based on the difference, the rod 310 and the stopper 330 of the pressure controller 300 may be moved and the spring 320 may be compressed.

As illustrated in FIG. 2, when the difference is less than or equal to a threshold pressure, a moving distance of the rod 310 and the stopper 330 of the pressure controller 300 may be relatively short and thus, a degree of deformation of the spring 320 may also be relatively small.

Accordingly, the stopper 330 may move not to pass by the inlet 200 formed on the one side of the inlet member 110, and thus, the inlet 200 may be maintained to be closed. Since the inlet 200 is closed, an external air may not enter the cupping cup 100.

From this, it may be indicated that the external air is not to flow into the cupping cup 100 and a sufficient or few amount of negative pressure is in the cupping cup 100.

It may also be indicated that a risk of an adverse effect such as blistering and bleeding occurring due to an excessive negative pressure in the cupping cup 100 is relatively low.

In an example of FIG. 3, the external pressure of the cupping cup 100 may be 1013 mmHg and the internal pressure of the cupping cup 100 may be 1013 - ooo mmHg. In this example, the difference between the internal pressure and the external pressure may be ooo mmHg. When the difference is greater than the threshold pressure, the moving distance of the rod 310 and the stopper 330 of the pressure controller 300 may be relatively long and thus, a degree of deformation of the spring 320 may also be relatively large.

Accordingly, the stopper 330 may move and pass by the inlet 200 formed on the one side of the inlet member 110 such that the rod 310 of the pressure controller 300 moves out of the through-hole 120 formed in the inlet member 110.

Also, the inlet 200 may be open, and the external air may flow into the cupping cup 100.

Through this, a negative pressure in the cupping cup 100 may decrease and the difference between the internal pressure and the external pressure of the cupping cup 100 may also decrease.

Thereafter, when at least a predetermined amount of air is absorbed in the cupping cup 100, an air inflow may be suspended due to the elasticity of the spring 320 and thus, a predetermined degree of pressure may be maintained in the cupping cup 100.

As such, in response to an operation of the pressure controller 300, the inlet 200 may be selectively open and closed to maintain a constant pressure in the cupping cup 100. Also, an adverse effect such as blistering and bleeding occurring due to an excessive negative pressure in the cupping cup 100 may be prevented.

Although a few embodiments of the present invention have been shown and described, the present invention is not limited to the described embodiments. Instead, it would be appreciated by those skilled in the art that changes may be made to these embodiments without departing from the principles and spirit of the invention, the scope of which is defined by the claims and their equivalents.

## Claims

1. A cupping device comprising:
a cupping cup attached to an area requiring a cupping therapy;
an inlet configured to allow communication between an inside and an outside of the cupping cup; and
a pressure controller configured to selectively open and close the inlet,
wherein an air inflow into the cupping cup is allowed or blocked based on a movement of the pressure controller.

2. The cupping device of claim 1, wherein the cupping cup includes an inlet member and the pressure controller is disposed in the inlet member.

3. The cupping device of claim 2, wherein the pressure controller includes:
a rod provided in a form of bar;
a spring elastically deformed and configured to cover the rod; and
a stopper attached to one end of the rod to open and close the inlet,
wherein the rod and the stopper are configured to move in the inlet member based on a difference between an internal pressure and an external pressure of the cupping cup.

4. The cupping device of claim 3, wherein the inlet member includes a through-hole of a size that does not allow the spring and the stopper to move out of the inlet member and allows the rod to move out of the inlet member.

5. The cupping device of claim 3, wherein the inlet is formed on a path along which the stopper moves in the inlet member so as to be open when the stopper passes by the inlet.

6. The cupping device of claim 3, wherein an elasticity of the spring corresponds to the difference between the internal pressure and the external pressure of the cupping cup, and a degree of deformation of the spring and a moving distance of the rod and the stopper change based on the difference.

7. The cupping device of claim 2, wherein the inlet member opens outward of the cupping cup to allow the air inflow.

8. A pressure control device for controlling an internal pressure of a case, the device comprising:
an inlet member attached on an inner side of the case and formed to have one open side to communicate with an outside of the case;
an inlet formed on one side of the inlet member;
a stopper configured to move in the inlet member to open and close the inlet; and
a spring disposed in the inlet member to be elastically deformed in response to a movement of the stopper.

9. The pressure control device of claim 8, further comprising:
a rod formed to have one side attached to the stopper and externally enclosed by the spring,
wherein a through-hole is formed in the inlet member through which the rod moves out of the inlet member.
